# EUROPEAN PATENT APPLICATION

(11) **EP 3 372 227 A1**
(43) Date of publication of application: **12.09.2018**
(21) Application number: 17460009.8
(22) Date of filing: 06.03.2017
(51) Int. Cl.: A61K 9/20, A61K 31/675

(54) **TABLET FORMULATION OF ADEFOVIR DIPIVOXIL**

(71) Applicant: MH10 Spolka z ograniczona odpowiedzialnoscia, 00-728 Warsaw (PL)
(72) Inventor: Kieronska, Hanna, 62-051 Wiry (PL); Berdzinska, Katarzyna, 62-095 Rakownia (PL); Palubicka, Anna, 64-500 Szamotuly (PL); Milewski, Marek, 62-030 Lubon (PL); Krause, Anna, 60-252 Poznan (PL); Kurek, Joanna, 01-046 Warszawa (PL); Rapacz, Joanna, 05-400 Otwock (PL)

(57) **Abstract**

The present invention relates to stable pharmaceutical tablet formulation of adefovir dipivoxil. Specifically, the invention relates to a tablet formulation, comprising pharmaceutically effective amount of adefovir dipivoxil crystalline form 1, characterized in that the tablet is prepared by direct compression method and comprises at least 75% of lactose monohydrate, 10% - 15% of starch, disintegrant and magnesium stearate.

## Description

### Technical Field

The present invention relates to pharmaceutical tablet formulation for human or veterinary use, which contains adefovir dipivoxil as an active substance.

### Background Art

Adefovir dipivoxil is the bis-pivaloyloxymethyl ester prodrug of the nucleotide analogue, adefovir. A structural modification of the parent drug has been carried out in order to increase the lipophilicity and to enhance the oral bioavailability of adefovir. Adefovir dipivoxil is a nucleotide reverse transcriptase inhibitor and exhibits a marked in vivo antiviral activity against Hepatitis type B virus (HBV). Adefovir and adefovir dipivoxil have been described, e.g., U.S. Patent Numbers 4,724,233 and 4,808,716, EP481214.

Different crystal and amorphous forms of adefovir dipivoxil are known. WO9904774 discloses crystalline adefovir dipivoxil form 1 and pharmaceutical composition comprising thereof. Crystalline form 1 of adefovir dipivoxil is anhydrous and contains little or no detectable amount of water. U.S. Patent No. 6,635,278 discloses an adefovir dipivoxil composition comprising an alkaline excipient to improve stability. In contact with moisture, adefovir dipivoxil is easily and rapidly hydrolysed and by-products of the hydrolysis accelerate further degradation of adefovir dipivoxil (Yuan et al., Pharm. Res. 17:1098, 2000).

The pharmaceutical composition of crystalline anhydrous adefovir dipivoxil has been sold on the market in a form of tablet under the trade name Hepsera, which contains 10 mg of adefovir dipivoxil. The process of preparation of Hepsera tablets utilizes wet granulation step and subsequent controlled drying process to reduce the water content of the granules. During further steps, the granules are blended together with excipients and compressed into tablet. Due to contact with water and elevated temperatures during wet granulation, it may be hard to avoid decomposition of adefovir dipivoxil. The main impurity found in commercial Hepsera, 10 mg, tablets is monopivaloyloxymethyl ester of adefovir. Monoester in comparison with diester is significantly less orally bioavailable. US patent application 5,663,159 A discloses that the relative oral bioavailability of the monopivaloyloxymethyl ester of adefovir in rats is only 6.5, which is even less than that of the original compound adefovir (7.8) and less than its diester, namely adefovir dipivoxil (17.0).

Summarizing, there is a need to develop a pharmaceutical formulation comprising adefovir dipivoxil with minimum degradation of the active substance.

### Summary of invention

The present invention relates to pharmaceutical tablet formulation of adefovir dipivoxil prepared by direct compression method and of improved stability of the active substance.

The present invention provides a tablet formulation of adefovir dipivoxil, wherein the tablet comprises pharmaceutically effective amount of adefovir dipivoxil crystalline form 1, at least 75% of lactose monohydrate, 10% - 15% of starch, disintegrant and magnesium stearate.

More particularly, the present invention provides pharmaceutical preparation of good stability in a form of tablet, prepared by direct compression method, wherein the tablet consists of 5% - 7% of adefovir dipivoxil crystalline form 1, at least 75% of lactose monohydrate, 10% - 15% of pregelatinised starch, 1% - 5% of disintegrant and 1% of magnesium stearate.

According to the preferred embodiment of the present invention disintegrant is selected from a group comprising: modified cellullose, modified starch, cross-linked polyvinylpyrrolidone, soy polysaccharides, cross-linked alginic acid, gellan gum, xantan gum, calcium silicate. Different types of disintegrants allow modification of dissolution profile. Superdisintegrants such as croscarmellose sodium gives very fast disintegration times of the tablets.

In a preferred embodiment of the present invention croscarmellose sodium is used as disintegrant.

In a preferred embodiment of the present invention the tablet formulation prepared by direct compression and comprising adefovir dipivoxil crystalline form 1 does not contain colloidal silicon dioxide.

In a preferred embodiment of the present invention the tablet formulation prepared by direct compression and comprising adefovir dipivoxil crystalline form 1 does not contain talc.

In the most preferred embodiment of the present invention one tablet consists of 10 mg of adefovir dipivoxil crystalline form 1, at least 75% of lactose monohydrate, 14% of pregelatinised maize starch, 3% of croscarmellose sodium and 1 % of magnesium stearate.

According to the preferred embodiment the tablet is prepared by direct compression method.

### Description of embodiments

During development of the generic tablet formulation containing 10 mg of adefovir dipivoxil the main goal was to obtain similar tablet characteristics to the commercial product Hepsera, 10 mg, tablets in terms of physical parameters (i.e. hardness, disintegration time), analytical parameters (dissolution profile) and of good stability. To minimise product degradation, direct compression was chosen for the development of the generic tablet formulation.

Initial attempt with generic formulation of adefovir dipivoxil was based on qualitative composition of the product Hepsera, which contains 10 mg of the active substance adefovir dipivoxil, pregelatinised starch, croscarmellose sodium, lactose monohydrate (in amount of 113 mg per tablet), talc and magnesium stearate. The blend with above presented composition was prepared, but has very poor flowability and it was not possible to conduct direct compression process. The main technological challenge was to improve flowability of powder blend. Poor flowability of the blend was partially caused by API specific physical properties (rough, abrasive particles).

In the second attempt talc was replaced by colloidal silicon dioxide, which improved flowability, but significant degradation of the active substance was observed during stability studies (formulation 2).

In another attempt starch was replaced with microcrystalline cellulose (direct compression suitable grade), but dissolution of the active substance from the prepared tablets was definitely too low.

When anhydrous lactose was used instead of lactose monohydrate (formulation 4) initial amount of impurities in the prepared tablets stored in accelerated degradation conditions was low, but after 42 days increased to 3.5% (as total impurities).

Surprisingly, it was found that eliminating colloidal silicon dioxide and using appropriate kind and amount of lactose (lactose monohydrate) gives blend, which can be directly compressed into tablets of improved stability (formulation 3, example 1).

The present inventors found that combination of appropriate kind of lactose (lactose monohydrate), pregelatinised starch, disintegrant and magnesium stearate in specific amounts, gives blend, which can be directly compressed into tablets of improved stability. When using croscarmellose sodium as a disintegrant, dissolution profile similar to Hepsera, 10 mg, tablets was obtained within the whole physiological pH range (from pH 1 to 6.8).

Stability of several types of tablet formulations comprising adefovir dipivoxil and commercial product Hepsera, 10 mg, tablets stored under accelerated storage conditions in 40°C and 75% relative humidity (RH) after given number of days is presented in Table 1. Percent amount of main degradation product, the monoester impurity being the pivaloyloxymethyl monoester of adefovir (adefovir pivoxil) was provided, together with total amount of impurities.

All tested tablets of formulations mentioned in tablet 1 have average weight of about 150 mg.

**Table 1**

| **Formulation** | **Impurities** | **Day 0** | **Day 7** | **Day 14** | **Day 42** |
|---|---|---|---|---|---|
| Hepsera 10 mg, tablets batch 1202937D | monoester impurity | 1.9% | NT | 2.2% | NT |
| | Total impurities | 2.8% | NT | 3.3% | NT |
| Formulation 2 | monoester impurity | 0.7% | 1.0% | 1.2% | 2.1% |
| | Total impurities | 0.9% | 1.2% | 1.5% | 2.4% |
| Formulation 3 | monoester impurity | 0.3% | 0.4% | 0.6% | 1.1% |
| | Total impurities | 0.4% | 0.6% | 0.8% | 1.3% |
| Formulation 4 | monoester impurity | 0.3% | 1.1% | 1.6% | 3.0% |
| | Total impurities | 0.4% | 1.3% | 1.8% | 3.5% |

| | | | | | |
|---|---|---|---|---|---|
| NT - not tested in the specified time point | | | | | |

Percent composition of the formulations mentioned in Table 1 is provided in Table 2.

**Table 2**

| **Composition** | **Hepsera, 10 mg, tablets** | **Formulation 2** | **Formulation 3** | **Formulation 4** |
|---|---|---|---|---|
| Adefovir dipivoxil | 6.67% | 6,67% | 6.67% | 6.67% |
| Lactose monohydrate | ~75% | 75.33% | 75.33% | NP |
| Lactose anhydrous | NP | NP | NP | 75.33% |
| Pregelatinised starch | unknown | 13.50% | 14.00% | 12.50% |
| Croscarmellose sodium | unknown | 3.00% | 3.00% | 3.00% |
| Talc | unknown | NP | NP | NP |
| Colloidal silicon dioxide | NP | 0.50% | NP | 1.50% |
| Magnesium stearate | unknown | 1.00% | 1.00% | 1.00% |

| | | | | |
|---|---|---|---|---|
| NP - not present | | | | |

The present invention is described in more details and specifically with references to the examples, which however are not intended to limit the present invention.

### Example 1

Common blend was prepared for a nominal batch size of 1500 g. Composition of the blend was as follows:

**Table 3**

| **Composition** | **% w/w** |
|---|---|
| Adefovir dipivoxil crystalline form 1 | 6.67% |
| Spray dried lactose monohydrate | 75.33% |
| Pregelatinised maize starch | 14.00% |
| Croscarmellose sodium | 3.00% |
| Magnesium stearate | 1.00% |

General manufacturing process was as follows:

All ingredients were weighed and sieved through 0.5 mm screen. Adefovir dipivoxil crystalline form 1, spray dried lactose monohydrate with 98% of particles having size of less than 250 µm (measured by air jet sieve), pregelatinised maize starch and croscarmellose sodium were added to low shear blender and were blended for 20 min. at 17 rpm. Magnesium stearate was then added and the mixture was blended for additional 5 minutes at 17 rpm. The prepared blend was used for direct compression on a rotatory press, with 7 mm punches. The average weigh of the single tablet obtained was 150 mg.

### Example 2

Common blend was prepared for a nominal batch size of 1500 g. Composition of the blend was as follows:

**Table 4**

| **Composition** | **% w/w** |
|---|---|
| Adefovir dipivoxil crystalline form 1 | 6.67% |
| Spray dried lactose monohydrate | 75.33% |
| Pregelatinised maize starch | 13.00% |
| Croscarmellose sodium | 4.00% |
| Magnesium stearate | 1.00% |

General manufacturing process was as follows:

All ingredients were weighed and sieved through 0.5 mm screen. Adefovir dipivoxil crystalline form 1, spray dried lactose monohydrate with 98% of particles having size of less than 250 µm and 50% of particles of the size less than 75 µm (measured by air jet sieve), pregelatinised maize starch and croscarmellose sodium were added to low shear blender and were blended for 20 min. at 17 rpm. Magnesium stearate was then added and the mixture was blended for additional 5 minutes at 17 rpm. The prepared blend was used for direct compression on a rotatory press, with 7 mm punches. The average weigh of the single tablet obtained was 150 mg.

### Example 3

Common blend was prepared for a nominal batch size of 2000 g. Composition of the blend was as follows:

**Table 5**

| **Composition** | **% w/w** |
|---|---|
| Adefovir dipivoxil crystalline form 1 | 5.0% |
| Spray dried lactose monohydrate | 81.75% |
| Pregelatinised maize starch | 10.0% |
| Croscarmellose sodium | 2.25% |
| Magnesium stearate | 1.00% |

General manufacturing process was as follows:

All ingredients were weighed and sieved through 0.5 mm screen. Adefovir dipivoxil crystalline form 1, spray dried lactose monohydrate with 98% of particles having size of less than 250 µm (measured by air jet sieve), pregelatinised maize starch and croscarmellose sodium were added to low shear blender and were blended for 20 min. at 17 rpm. Magnesium stearate was then added and the mixture was blended for additional 5 minutes at 17 rpm. The prepared blend was used for direct compression on a rotatory press, with 7 mm punches. The average weigh of the single tablet obtained was 200 mg.

## Claims

1. A tablet formulation, comprising pharmaceutically effective amount of adefovir dipivoxil crystalline form 1, **characterized in that** the tablet is prepared by direct compression method and comprises at least 75% of lactose monohydrate, 10% - 15% of starch, disintegrant and magnesium stearate.

2. A tablet formulation according to claim 1, **characterized in that** it comprises 10 mg of adefovir dipivoxil.

3. A tablet formulation according to claim 1, comprising pregelatinised maize starch.

4. A tablet formulation according to claim 1, wherein croscarmellose sodium is used as disintegrant.

5. A tablet formulation according to claim 1, **characterized in that** it comprises 1% - 5% of disintegrant.

6. A tablet formulation according to claim 1, comprising spray dried lactose monohydrate.

7. A tablet formulation according to claim 1, **characterized in that** a tablet consists of adefovir dipivoxil crystalline form 1 in amount of 5% - 7%, at least 75% of lactose monohydrate, 10% - 15% of pregelatinised starch, 1% - 5% of disintegrant and 1 % of magnesium stearate.

8. A tablet formulation according to claim 1, **characterized in that** a tablet consists of 10 mg of adefovir dipivoxil crystalline form 1, at least 75% of lactose monohydrate, 14% of pregelatinised maize starch, 3% of croscarmellose sodium and 1 % of magnesium stearate.

9. The tablet formulation according to any preceding claim, **characterized in that** it does not contain talc.

10. The tablet formulation according to any preceding claim, **characterized in that** it does not contain colloidal silicon dioxide.
